# EUROPÄISCHE PATENTSCHRIFT

(11) **EP 1 000 122 B1**
(45) Veröffentlichungstag und Bekanntmachung des Hinweises auf die Patenterteilung: **23.10.2002**
(21) Anmeldenummer: 98947330.1
(22) Anmeldetag: 21.07.1998
(51) Int. Cl.: C08L 93/02, C08J 3/05

(54) **WÄSSRIGE SCHELLACKLÖSUNG ODER -DISPERSION**
AQUEOUS SHELLAC SOLUTION OR DISPERSION
SOLUTION OU DISPERSION AQUEUSE DE SHELLAC

(30) Priorität: 01.08.1997 DE 19734548
(43) Veröffentlichungstag der Anmeldung: 17.05.2000
(73) Patentinhaber: LANCASTER GROUP GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE9802088
(87) Internationale Veröffentlichungsnummer: WO99006488

(56) Entgegenhaltungen:
- WO-A-95/02339
- US-A- 2 834 770
- US-A- 3 261 705

## Beschreibung

Die Erfindung betrifft eine wäßrige Lösung oder Dispersion mit hohen Schellackgehalten.

Schellack ist bekanntlich ein natürliches Harz, das von der weiblichen Lackschildlaus Kerria lacca ausgeschieden wird. Das für kosmetische Zwecke eingesetzte, gereinigte, wachsfreie Harz, das ein Molekulargewicht von etwa 1000 hat, besteht aus einer Reihe von Hydroxymonodicarbonsäuren in Form von Lactonen, Lactiden und intramolekularen Estern. Die Hauptkomponenten sind Aleuritinsäure, Schellolsäure und Jalarsäure (C₁₅H₂₀O₅) mit Molgewichten von 304, 296 bzw. 280. Schellack ist daher eher ein Oligomeres von Hydroxycarbonsäuren als ein Polymeres. Mittels eines Lösungsmittelextraktionsverfahrens z.B. mit einem Alkohol, können Verunreinigungen entfernt werden, und nach Entfärbung mit Aktivkohle und Verdampfen des Alkohols kann Schellack in einen gelblichen bis schwach gelblichen Feststoff überführt werden.

Aus der WO96/41630 ist bekannt, in einem aus drei oder mehr Schichten gebildeten Lichtschutzmittel Schellack als Trennmittel zwischen den voneinander abgesetzten Schichten zu verwenden, so daß sich ein Mehrschichtaufbau des Lichtschutzmittels auf der Haut ausbildet, und die Haut nur mit der Schicht in Kontakt kommt, die hautverträgliche anorganische Lichtschutzmittel enthält.

Schellacklösungen oder -dispersionen auf wäßriger Basis sind bisher nicht bekannt.

Der Erfindung liegt daher die Aufgabe zugrunde, wäßrige Lösungen oder Dispersionen mit Schellack zu entwickeln, insbesondere solche mit höheren Schellackgehalten und höheren Viskositäten als etwa 200 mPa·s.

Erfindungsgemäß bereitgestellt wird eine wäßrige Schellacklösung oder -dispersion, gekennzeichnet durch einen Gehalt von:
Schellack von 1 bis 60 Gew-%,
wenigstens ein wasserlöslicher Filmbildner von 0,1 bis 3 Gew-%,
wenigstens ein im sauren pH-Bereich beständiger Gelbildner von 0,1 bis 1 Gew-%,
Wasser bis 100 %,
und einen pH-Wert von 2 bis 4,2.

Alle Prozentangaben sind auf das Gewicht (die Masse) der Gesamtdispersion oder der Lösung bezogen.

Besonders bevorzugte Gehalte von Schellack liegen im Bereich von 10 bis 50 Gew-%, vorzugsweise 15 bis 50 Gew-% und insbesondere bei 20 bis 40 Gew-%.

Die Schellacklösung oder -dispersion ist nur im sauren Bereich beständig, insbesondere im pH-Bereich von 2 bis 3,5. Die Einstellung des pH-Wertes kann mit einer beliebigen anorganischen oder organischen Säure oder einem stark sauren Salz erfolgen, beispielsweise mit Citronensäure. Dabei sollte jedoch die Wahl der Säure oder des Salzes dem Verwendungszweck des Schellacks angepaßt sein.

Ein weiterer Gegenstand der Erfindung ist ein Verfahren zur Herstellung von wäßrigen Schellacklösungen oder -dispersionen. Das Verfahren besteht darin, daß in eine wäßrige Lösung, die einen pH-Wert von 2 bis 4,2 hat und wenigstens einen wasserlöslichen Filmbildner sowie wenigstens einen im sauren pH-Bereich beständigen Gelbildner enthält, bei einer Temperatur von 5 bis 20° C teilchenförmiger Schellack unter Rühren bei 10 bis 1500 U/Min eingetragen und homogenisiert wird.

Die neuen Schellacklösungen liegen bei einem Schellackgehalt bis etwa 30 Gew-% vor und weisen in diesem Bereich Viskositäten bis zu zirka 2500 mPa·s auf. Beispielsweise hat eine 10 %ige Schellacklösung eine Viskosität von etwa 500 mPa·s, eine 20 %ige Schellacklösung von etwa 1000 mPa·s.

Oberhalb etwa 30 % Schellack ergeben sich wäßrige Dispersionen mit guter Dispergierbarkeit des teilchenförmigen Schellacks bis etwa 60 Gew-%.

Schellacklösungen können durch Zugabe von z. B. Phospholipiden in eine Dispersion umgewandelt werden, so daß im gesamten beanspruchten Bereich auch Dispersionen vorliegen können.

Wesentlich für die Herstellung der Dispersion und der Lösung von Schellack in Wasser ist der pH-Wert von etwa 2 bis 4,2 und die Anpassung der Oberflächenspannung der einzubringenden Komponenten an die Oberflächenspannung von Wasser.

Als wasserlöslicher Filmbildner kann vorteilhaft ein Polyvinylpyrrolidon eingesetzt werden, z. B. PCPK30®. Weitere mögliche Filmbildner sind Chitosan, mikrokristallines chitosan, quarterniertes Chitosan, Polymere der Acrylsäurerreihe, quarternäre Cellulosederivate, Hyaluronsäure und deren Salze.

Als im sauren pH-Bereich beständigen Gelbildner kann z. B. ein modifiziertes natürliches Polysaccharid wie Guar Hydroxypropyltrinonium Chloride (CTFA-Name) (Jaguar C14S®) verwendet werden. Weitere mögliche Gelbildner sind Agar, Alginate, Alginsäure.

Auch Gemische mehrerer Filmbildner und/oder Gelbildner können verwendet werden.

Vorteilhaft wird in dem erfindungsgemäßen Verfahren Schellack eingesetzt, der gereinigt und entwachst ist und ein Molekulargewicht von 1000 bis 1010 hat. Der pH-Wert liegt vorzugsweise bei 3,5 bis 2 und die Temperatur im Bereich von 5 bis 15 °C.

Ein weiteres Merkmal der Erfindung ist die Abhängigkeit der Viskosität der Schellacklösung oder -dispersion von der Konzentration des Schellacks, wie bereits oben ausgeführt. Bevorzugt sind daher Schellacklösungen oder -dispersionen im Bereich von 200 bis 2500 mPa·s, insbesondere 500 bis 2500 mPa·s. Ein besonders bevorzugter Bereich liegt zwischen 1000 und 2000 mPa·s. Mit Konzentrationen von etwa 55 bis 60 % bei den neuen wasserlöslichen Schellackdispersionen werden Viskositäten von etwa 100000 mPa·s erreicht. Das bedeutet, daß die hochkonzentrierten Dispersionen von etwa 45 bis 60 Gew-% cremig bis pastenartig sind, sich jedoch auch in dieser Form gut verarbeiten lassen.

Die erfindungsgemäße Lösung oder Dispersion kann als Anstrichmittel auf Wasserbasis (Lack), in der Lebensmittel industrie bei der Herstellung von Schokolade, Bonbons usw. verwendet werden. Sie kann außerdem in kosmetischen Emulsionen oder Gelen eingesetzt werden, z. B. zur Verbesserung der Wasserbeständigkeit derartiger Produkte.

Die Erfindung soll nachstehend durch Beispiele näher erläutert werden. Alle Prozentangaben sind auf das Gewicht (Masse) bezogen.

### Beispiel 1

In 100 ml Wasser wurde unter Rühren der pH-Wert mit citronensäure auf 3,3 eingestellt. Danach wurde 1,3 g Polyvinylpyrrolidon (PVPK30®) und 0,6 g Guar Hydroxypropyltrimonium Chloride (CTFA-Name) (Jaguar C14S®) unter weiterem Rühren hinzugegeben. In die erhaltene Lösung wurden 15 g gereinigter, entwachster, teilchenförmiger Schellack bei etwa 13-15 °C und bei weniger als 1000 U/Min. eingetragen und die Lösung bei 1450 U/Min. homogenisiert. Die auf diese Weise hergestellte Schellacklösung hatte eine Viskosität von 540 mPa·s.

### Beispiel 2

Es wurde wie im Beispiel 1 gearbeitet, mit Ausnahme dessen, daß der pH-Wert 2,8 betrug und 33 g Schellack eingetragen wurden. Man erhielt eine Lösung mit einer Viskosität von 1720 mPa·s.

### Beispiel 3

Es wurde wie im Beispiel 1 verfahren, mit Ausnahme dessen, daß 2,2 g PVPK, 0,75 g Jaguar C14S eingesetzt, der pH-Wert auf 3,1 eingestellt wurde und 53 g Schellack eingetragen wurden. Man erhielt eine pastenartige Dispersion mit einer Viskosität von 92.500 mPa·s.

## Patentansprüche

1. Wäßrige Schellacklösung oder -dispersion, **gekennzeichnet durch** einen Gehalt an
Schellack von 1 bis 60 Gew-%,
wenigstens einem wasserlöslichen Filmbildner von 0,1 bis 3 Gew-%,
wenigstens einem in sauren pH-Bereich beständigen Gelbildner von 0,1 bis 1 Gew-%,
Wasser bis 100 Gew-%,
und einen pH-Wert von 2 bis 4,2.

2. Schellacklösung oder -dispersion nach Anspruch 1, **dadurch gekennzeichnet, daß** der Gehalt an Schellack im Bereich von 10 bis 50 Gew-% liegt.

3. Schellacklösung oder -dispersion nach Anspruch 2, **dadurch gekennzeichnet, daß** der Gehalt an Schellack im Bereich von 15 bis 50 Gew-% liegt.

4. Schellacklösung oder -dispersion nach Anspruch 2, **dadurch gekennzeichnet, daß** der Gehalt an Schellack im Bereich von 20 bis 40 Gew-% liegt.

5. Schellacklösung oder -dispersion nach Anspruch 1, **dadurch gekennzeichnet, daß** der pH-Wert 2 bis 3,5 beträgt.

6. Schellacklösung oder -dispersion nach Anspruch 1, **dadurch gekennzeichnet, daß** die Lösung oder Dispersion eine Viskosität von 200 bis 2500 mPa·s hat.

7. Schellacklösung oder -dispersion nach Anspruch 1, **dadurch gekennzeichnet, daß** die Lösung oder Dispersion eine Viskosität von 500 bis 2500 mPa·s hat.

8. Schellacklösung oder -dispersion nach Anspruch 7, **dadurch gekennzeichnet, daß** die Lösung oder Dispersion eine Viskosität im Bereich von 1000 bis 2000 mPa·s hat.

9. Verfahren zur Herstellung einer wäßrigen Schellacklösung oder -dispersion, **dadurch gekennzeichnet, daß** man in eine wäßrige Lösung, die einen pH-Wert von 2 bis 4,2 hat und wenigstens einen wasserlöslichen Filmbildner und wenigstens einen im sauren pH-Bereich beständigen Gelbildner enthält, bei einer Temperatur von 5 bis 20° C teilchenförmigen Schellack unter Rühren bei 10 bis 1500 U/Min einträgt und homogenisiert.

10. Verfahren nach Anspruch 9, **dadurch gekennzeichnet, daß** man einen Schellack einsetzt, der gereinigt und entwachst ist und ein Molekulargewicht von 1000 bis 1010 hat.

11. Verfahren nach Anspruch 9, **dadurch gekennzeichnet, daß** der pH-Wert im Bereich von 3,5 bis 2 gehalten wird.

12. Verfahren nach Anspruch 9, **dadurch gekennzeichnet, daß** man den teilchenförmigen Schellack bis zum Erreichen einer Viskosität der Lösung von 200 bis 2500 mPa·s einträgt.

13. Verfahren nach Anspruch 9, **dadurch gekennzeichnet, daß** als Gelbildner Guar Hydroxypropyltrimonium Chloride hinzugegeben wird.

14. Verfahren nach Anspruch 9, **dadurch gekennzeichnet, daß** man die Temperatur im Bereich von 5 bis 15° C hält.

## Claims

1. An aqueous shellac solution or dispersion, **characterised by** a content of
Shellac of 1 to 60 %wt;
at least one water-soluble film former of 0.1 to 3 %wt;
at least one gel former, resistant in the acid pH range, of 0.1 to 1 %wt;
water to 100 % weight; and
a pH value of 2 to 4.2.

2. A shellac solution or dispersion according to claim 1, **characterised in that** the content of shellac ranges from 10 to 50 %wt.

3. A shellac solution or dispersion according to claim 2, **characterised in that** the content of shellac ranges from 15 to 50 %wt.

4. A shellac solution or dispersion according to claim 2, **characterised in that** the content of shellac ranges from 20 to 40 %wt.

5. A shellac solution or dispersion according to claim 1, **characterised in that** the pH value ranges from 2 to 3.5.

6. A shellac solution or dispersion according to claim 1, **characterised in that** the viscosity of the solution or dispersion ranges from 200 to 2,500 mPa·s.

7. A shellac solution or dispersion according to claim 1, **characterised in that** the viscosity of the solution or dispersion ranges from 500 to 2,500 cP·s.

8. A shellac solution or dispersion according to claim 7, **characterised in that** the viscosity of the solution or dispersion ranges from 1,000 to 2,000 mPa·s.

9. A method for producing an aqueous shellac solution or dispersion, **characterised in that** particle-shaped shellac is added to an aqueous solution and homogenised by stirring at 10 to 1,500 rev/min at a temperature of 5 to 20 °C; said aqueous solution having a pH value of 2 to 4.2 and containing at least one water-soluble film former and at least one gel former which is resistant in the acid pH range.

10. A method according to claim 9, **characterised in that** a purified and dewaxed shellac is used with a molecular weight of 1,000 to 1,010.

11. A method according to claim 9, **characterised in that** the pH value is kept in the range between 3.5 and 2.

12. A method according to claim 9, **characterised in that** the particle-shaped shellac is added until the viscosity of the solution is between 200 and 2,500 mPa·s.

13. A method according to claim 9, **characterised in that** guar hydroxypropyltrimonium chloride is added as a gel former.

14. A method according to claim 9, **characterised in that** the temperature is kept in the range of 5 to 15 °C.

## Revendications

1. Solution ou dispersion aqueuse de shellac, **caractérisée par** une teneur en
shellac allant de 1 à 60 % en poids,
au moins un filmogène hydrosoluble allant de 0,1 à 3 % en poids,
au moins un gélifiant stable dans la plage de pH acide allant de 0,1 à 1 % en poids,
eau jusqu'à 100 %,
et par un pH de 2 à 4,2.

2. Solution ou dispersion aqueuse de shellac selon la revendication 1, **caractérisée en ce que** la teneur en shellac se situe dans une plage allant de 10 à 50 % en poids.

3. Solution ou dispersion aqueuse de shellac selon la revendication 2, **caractérisée en ce que** la teneur en shellac se situe dans une plage allant de 15 à 50 % en poids.

4. Solution ou dispersion aqueuse de shellac selon la revendication 2, **caractérisée en ce que** la teneur en shellac se situe dans une plage allant de 20 à 40 % en poids.

5. Solution ou dispersion aqueuse de shellac selon la revendication 1, **caractérisée en ce que** le pH atteint 2 à 3,5.

6. Solution ou dispersion aqueuse de shellac selon la revendication 1, **caractérisée en ce que** la solution ou dispersion a une viscosité d'environ 200 à 2500 mPa·s.

7. Solution ou dispersion aqueuse de shellac selon la revendication 1, **caractérisée en ce que** la solution ou dispersion a une viscosité d'environ 500 à 2500 mPa·s.

8. Solution ou dispersion aqueuse de shellac selon la revendication 7, **caractérisée en ce que** la solution ou dispersion a une viscosité d'environ 1000 à 2000 mPa·s.

9. Procédé de préparation d'une solution ou dispersion de shellac, **caractérisé en ce que** l'on incorpore et homogénéise du shellac particulaire à une température de 5 à 20 °C en agitant à environ 10 à 1500 tours/min dans une solution aqueuse qui a un pH de 2 à 4,2 et qui contient au moins un gélifiant stable dans la zone de pH acide.

10. Procédé selon la revendication 9, **caractérisé en ce que** l'on met en oeuvre du shellac qui est purifié et déciré et a un poids moléculaire de 1000 à 1010.

11. Procédé selon la revendication 9, **caractérisé en ce que** l'on maintient le pH dans la zone allant de 3,5 à 2.

12. Procédé selon la revendication 9, **caractérisé en ce que** l'on incorpore le shellac particulaire jusqu'à obtention d'une viscosité de la solution allant de 200 à 2500 mPa·s.

13. Procédé selon la revendication 9, **caractérisé en ce que** l'on ajoute comme gélifiant du Guar Hydroxypropyltrimonium Chloride.

14. Procédé selon la revendication 9, **caractérisé en ce que** l'on maintient une température dans la zone allant de 5 à 15°C.
